## Europäisches Patentamt

## European Patent Office

## Office européen des brevets

(11) Publication number: **0 170 668**
**B1**

# (12) EUROPEAN PATENT SPECIFICATION

(45) Date of publication of patent specification: **08.08.90**

(21) Application number: **85900656.1**

(22) Date of filing: **25.01.85**

(86) International application number:
**PCT/DK85/00004**

(87) International publication number:
**WO 85/03234 01.08.85 Gazette 85/17**

(51) Int. Cl.⁵: **A 61 N 1/32**

(54) AN APPARATUS FOR TREATING SCARS.

(30) Priority: **26.01.84 DK 369/84**

(43) Date of publication of application:
**12.02.86 Bulletin 86/07**

(45) Publication of the grant of the patent:
**08.08.90 Bulletin 90/32**

(84) Designated Contracting States:
**AT DE FR GB SE**

(56) References cited:
**US-A-3 911 930**
**US-A-3 918 459**
**US-A-4 019 510**
**US-A-4 372 319**

**Derwent's abstract No. 4992 E/24, SU 858 850**
**Derwent's abstract No. 84-235771/38, SU 1 068 127 A**

(73) Proprietor: **KRISTENSEN, Jorn**
**Sneppevej 28**
**DK-7600 Struer (DK)**

(72) Inventor: **KRISTENSEN, Jorn**
**Sneppevej 28**
**DK-7600 Struer (DK)**

(74) Representative: **Dietz, Frans Anton et al**
**Vereenigde Octrooibureaux Nieuwe Parklaan**
**107 P.O. Box 87930**
**NL-2508 DH Den Haag (NL)**

Courier Press, Leamington Spa, England.

## Description

The present invention relates to an apparatus for treating scars, comprising a stable current generator connected to a pair of plate electrodes.

From US—A—4,019,510 is known a method for treating skin ulcers, infections and the like by electrotherapy. Here is used a direct current generator, the polarity of which is reversed from time to time, such that a very low intensity direct current of +4 µA during 50 minutes is followed by a direct current of −250 µA during 5 minutes. With this polarity reversal, the depositing of unwanted material on the electrodes is prevented without influencing the favourable effect of the electro-therapeutic treatment. This known apparatus works at a DC-voltage of 4.8 volt.

The invention has for its purpose to provide an apparatus for treating scars by means of which may be obtained an effective and more purposeful regeneration of cicatricial tissue—including also a regeneration of the underlying skin layer of the scar.

Thereto, according to the invention the apparatus of the above-mentioned type is characterized in that the generator generates a train of current pulses, the width of the current pulses being in the range 1—2 ms, the spacing of the current pulses being in the range 2—4 ms, and in each pulse train the current level gradually rising from 0 mA to reach an end value of about 30 mA.

Hereby corresponding current pulses are generated in and around the scar and a DC-current is generated in the subcutis which in addition to the current pulse stimulation also is warmed-up by the DC-effect.

In order to provide a good current dispersion between the electrodes and the skin, to avoid uncontrollable current condition and over-heating of the skin which by way of example otherwise may occur if the electrodes cover the particular sensitive acupuncture points ("hot spots"), the surface area of each of the electrodes is preferably 50—100 cm$^2$.

In order to allow the accumulation of energy in the treatment area, the apparatus according to the invention comprises a diode coupled between the current generator and one of the electrodes.

The invention is described in more detail in the following, reference being made to the accompanying drawing, in which:

Fig. 1 shows an embodiment of an apparatus according to the invention;

Fig. 2 shows a schematical diagram of a circuit comprising the apparatus of Fig. 1 connected to an electronic "model" of the skin;

Fig. 3 shows a measured voltage graph at the beginning of a treatment, while

Fig. 4 shows a correspondingly measured voltage graph representing a longer period of time.

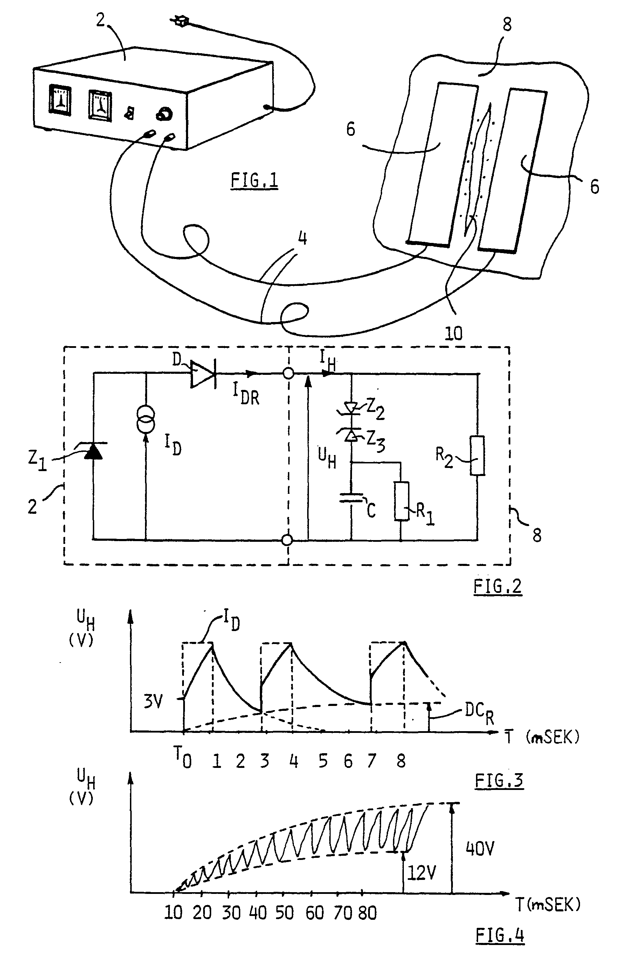

The apparatus shown in Fig. 1 consists of a micro processor controlled current generator which via terminal conductors 4 is connected with plate shaped aluminium electrodes 6 positioned on the skin 8 at opposite sides of an elongated operation scar 10. Of course the electrodes have to be adjusted in accordance with the actual treatment (the size and shape of the scar), and preferably be as big as possible and normally from 50—100 cm$^2$.

In order to obtain a uniform current density between the skin and the electrodes, use is made of a dispersing medium suspended in a suctorial material layer such as viscose (cotton wool). As dispersing medium may be used ordinary, calcareous water.

The current pulse treatment takes place via the plate shaped aluminium electrodes 6, which in the treating solution are positioned on the skin with an intermediary layer of viscose in which the dispersion medium which shall ensure a uniform current distribution and cooling of the skin is dispersed.

The current generator has the following data:

Short-circuit current (i.e. the current without loading between the skin and electrodes): Max. 88 mA.

No-load voltage: 60 volt.

Working voltage: 25—40 volt.

Working current: 25—35 mA.

Electrode conductance: 50—60 mS/m$^2$.

Dispersion medium: Calcareous water, i.e. hard water with the following specification: Hardness 8—9; Conductance 0.5—0.7 mS; pH-value 7—8; Chloride (Cl-) 2.5—3.5 mg/l; Potential −40 to −100 mV.

The skin "model" shown in Fig. 2 is adapted to connect directly between the terminal conductors 4, i.e. *without* dispersion medium.

Specification of the circuit shown in Fig. 2:

$Z_1$=Zener diode with tipping voltage of 60 volt.

$I_D$=0—40 mA.

D=barrage diode.

$Z_2$ and $Z_3$=backcoupled Zener diodes, tipping voltage 2.7 volt.

$C_1$=680 nF.

$R_1$=4.7 Kohm.

$R_2$=10 Kohm.

By measuring is ascertained that energy may be accumulated in the tissue. The charging is built up with 25 mA in 0.5—1 ms and is discharged again in typically 2—4 ms. The skin cells may thus have a natural voltage potential (Zener function) and a capacitive electrolyte.

The treating time is normally about 20 minutes and the treating frequency is normally 2 days. Ordinary results are obtained after 4—6 treatments as the scar begins to tickle and the scar begins to suppurate particularly from the edge area thereof, which lasts about 8 days. The visual effect moreover appears by the scar shrinking in the width and by the normally light and parchment-like skin of the scar again getting its normal blushing and suppleness.

It is not known for certain why the treatment has this very favourable result, but probably there appears by the combined electro-stimulation and warming treatment a beneficial influence of the immune system which is suppurated by the said suppurating of lymph from the scar edge. After about 18 treatments, the maximal effect seems to be obtained, where the scar can hardly be seen. The skin of the scar has again become soft and supple, i.e. that even the natural collagen of the skin seems to have been regenerated. Besides it seems as if the subcutis is reestablished including the fine netting of blood vessels and lymphatics. Even the pigmentation of the subcutis seems to be regenerated, because the skin again is sensitive to sun blushing.

By experiments with the apparatus according to the invention in established hospital departments, the following theories regarding the function of the treatment are used:

Theory 1: The metabolic model

A cell voltage has a voltage potential in relation to the tissue fluid of 10—100 mV depending of the cell type. The potential comes into being partly by diffusion and osmosis and by actively pumping out of potassium ions (K+) to the tissue fluid through the cell membrane. By applying a static field given by a constant current in the tissue, sodium ions (Na+) and chloride ions (Cl−) are moved to the negative and positive electrode, respectively. Since there now becomes deficit of sodium ions (Na+) in the tissue fluid, more ions are drained off into the tissue fluid. The cell becomes active to calcium ions (Ka++) which are attracted towards the cell membrane and release regeneration mechanisms. Possibly the tissue hormone histamine is released out from the mast cells of the connective tissue and from the granular of the cells. Hereby the cell production is stimulated by cyclic AMP, which then activates the enzyme apparatus of the cell.

Theory 2: Electro-dynamic stimulation

It has been known for a long time that calcium ions have a powerful influence on the function of the cell membrane and play an important part in the excitation of the nerve impulses. Therefore there are raised theories about calcium ions being bound to proteins which are bound to the cell membrane and here act as ion gates against potassium ions and chloride ions, i.e. have a nonlinear characteristic, which release large energy amounts by little stimuli. ELF (extremely low frequency <300 Hz) will be able to beat calcium ions loose from the linkages like dust from a vibration surface. This function will give a mechanism which will form resonance with a Q greater than 1 and transmit the field mechanically into the large DNA-molecules of the cell nucleus.

## Claims

1. An apparatus for treating scars, comprising a stable current generator (2) connected to a pair of plate electrodes (6); characterized in that the generator generates a train of current pulses, the width of the current pulses being in the range 1—2 ms, the spacing of the current pulses being in the range 2—4 ms, and in each pulse train the current level gradually rising from 0 mA to reach an end value of about 30 mA.

2. Apparatus according to claim 1, characterized in that the surface area of each of the electrodes is 50—100 cm².

3. Apparatus according to claim 1 or 2, characterized in that a diode is coupled between one of the electrodes and the corresponding output connection of the current generator.

## Patentansprüche

1. Vorrichtung zur Behandlung von Schrammen, versehen mit einem mit einem Plattenelektrodenpaar (6) verbundenen Stabilstromerzeuger (2), dadurch gekennzeichnet, daß der Erzeuger eine Reihe von Stromimpulsen erzeugt, wobei die Breite der Stromimpulse 1—2 ms ist, der Abstand zwischen den Stromimpulsen 2—4 ms ist und in jeder Impulsreihe das Stromniveau allmählich von 0 mA ansteigt, um einen Endwert von ungefähr 30 mA zu erreichen.

2. Vorrichtung nach Anspruch 1, dadurch gekennzeichnet, daß die Elektroden je eine Oberfläche von 50—100 cm² haben.

3. Vorrichtung nach Anspruch 1 oder 2, dadurch gekennzeichnet, daß zwischen einer der Elektroden und der entsprechenden Ausgangsverbindung des Stromerzeugers eine Diode gekoppelt ist.

## Revendications

1. Appareil de traitement de cicatrices, comprenant un générateur (2) de courant stable connecté à une paire des electrodes (6) en forme de plaque, caractérisé en ce que le générateur engendre un train des impulsions de courant, la largeur des impulsions de courant étant dans la région 1—2 ms, l'espace entre les impulsions de courant étant dans la région 2—4 ms, et dans chaque train des impulsions le niveau de courant montant graduellement de 0 mA pour atteindre une valeur finale d'environ de 30 mA.

2. Appareil selon la revendication 1, caractérisé en ce que l'aire de la surface de chacune des electrodes est 50—100 cm².

3. Appareil selon la revendication 1 ou 2, caractérisé en ce que une diode est reliée entre une des electrodes et la borne de sortie correspondant du générateur de courant.

FIG.1

FIG.2

FIG.3

FIG.4